# EUROPÄISCHE PATENTSCHRIFT

(11) **EP 3 153 115 B1**
(45) Veröffentlichungstag und Bekanntmachung des Hinweises auf die Patenterteilung: **04.07.2018**
(21) Anmeldenummer: 16002062.4
(22) Anmeldetag: 23.09.2016
(51) Int. Cl.: A61B 17/16

(54) **KNOCHENSTANZE MIT UNVERLIERBAREM STANZSCHIEBER**
BONE PUNCH WITH CAPTIVE PUNCHING SLIDE
KERRISON RONGEUR AVEC COULISSEAU D'ESTAMPAGE IMPERDABLE

(30) Priorität: 29.09.2015 DE 202015006833 U
(43) Veröffentlichungstag der Anmeldung: 12.04.2017
(73) Patentinhaber: Zepf, Christoph, 78589 Dürbheim (DE)
(72) Erfinder: Schreider, Sergej, 78628 Rottweil (DE)
(74) Vertreter: Binner, Bernhard

(56) Entgegenhaltungen:
- DE-U1-202004 017 974

## Beschreibung

Die Erfindung betrifft eine Knochenstanze mit einem Grundkörper, der aus einem Führungsschaft, einem am Führungsschaft feststehend und abgewinkelt angeordneten Handgriff besteht und mit einem Stanzschieber, welcher auf einer dem Handgriff gegenüber liegenden, oberen Führungsfläche des Führungsschaftes längs des Führungsschaftes verstellbar aufliegt und mit einem Betätigungshebel, welcher in einem Verbindungsbereich des Handgriffes zum Führungsschaft zwischen zwei voneinander beabstandeten Seitenwänden schwenkbar gelagert ist, wobei der Betätigungshebel einen Stellhebel bildet, über welchen der Betätigungshebel mit dem Stanzschieber zu dessen Verstellung längs des Führungsschaftes entgegen der Federkraft einer Rückstellfeder in Wirkverbindung steht und wobei der Stanzschieber über T-förmig profilierte, formschlüssig ineinander greifende und durch Längsverschiebung lösbare Führungselemente am Führungsschaft längs verstellbar geführt ist.

Aus der DE 20 2004 017 974 U1 ist eine als chirurgisches Stanzinstrument bezeichnete Knochenstanze der gattungsgemäßen Art bekannt. Diese bekannte Knochenstanze weist einen Führungsschaft auf, an welchem ein Handgriff feststehend angeordnet ist, welcher abgewinkelt zum Führungsschaft verläuft. Im Übergangsbereich vom Führungsschaft zu diesem Handgriff ist des Weiteren ein Betätigungshebel vorgesehen, welcher schwenkbar in diesem Verbindungsbereich gelagert ist. In diesem Verbindungsbereich bzw. im Bereich der Schwenklagerung des Betätigungshebels, bildet der Verbindungsbereich zwei voneinander beabstandete Seitenwände, zwischen welchen der Betätigungshebel mit einem Stellhebel hindurch ragt und oberseitig die ebene Führungsfläche des Führungsschaftes im normalen Betriebszustand überragt.

Dieser Stellhebel des Betätigungshebels steht dabei mit einer zum Führungsschaft hin, nach unten offenen Aussparung eines Stanzschiebers in Wirkverbindung. Der Stanzschieber weist eine zum Stanzschieber hin eine "untere" Gleitfläche auf, mit welcher der Stanzschieber längsverschiebbar auf dem Führungsschaft angeordnet ist. Zur unverlierbaren Halterung des Stanzschiebers auf dem Führungsschaft sind T-förmig profilierte, formschlüssig ineinander greifende Führungselemente vorgesehen. Der Betätigungshebel kann aufgrund einer speziellen Konstruktion der Lagerung des Betätigungshebels zwischen den beiden Seitenwänden aus seiner aktiven, mit seinem Stellhebel mit dem Stanzschieber in Eingriff stehenden Position "zurückgezogen" werden, so dass der Stellhebel nicht mehr mit dem Stanzschieber in Wirkverbindung steht. In dieser "zurück gezogenen" Position des Betätigungshebels kann der Stanzschieber distal soweit entlang des Führungsschaftes zurückgestellt werden, bis die Führungselemente des Stanzschiebers und des Führungsschaftes außer Eingriff gelangen. In dieser Position ist somit der Stanzschieber in einfacher Weise vollständig vom Führungsschaft abnehmbar, da keinerlei Verbindung mehr zwischen diesen Teilen besteht. Der Führungsschaft, der Verbindungsbereich und der Handgriff bilden dabei ein Art Grundkörper.

Dabei geht der Gegenstand der DE 20 2004 017 974 U1 von chirurgischen Stanzinstrumenten nach der DE 43 16 768 A1, DE 43 16 769 C1, DE 295 00 422 U1, US 5 026 375, US 5 273 519, US 5 569 258 sowie der WO 96/39 959 aus.

Bei diesen Knochenstanzen bzw. Stanzinstrumenten sind die Rückstellfedern für den Betätigungshebel blattfederartig ausgebildet und wirken zwischen dem feststehenden Handgriff und dem Betätigungshebel als Rückstellfeder. Da bei diesen bekannten Rückstellfedersystemen die Montage und Demontage beschwerlich ist, können solche Rückstellfedern die Handhabung eines solchen Stanzinstrumentes bzw. einer solchen Knochenstanze erschweren, da diese Federn erlahmen können oder nicht die erforderliche Rückstellkraft aufbringen, um ein einwandfreies Funktionieren des Stanzinstrumentes zu gewährleisten. Des Weiteren sind diese Rückstellfedern nicht ohne Weiteres voneinander bzw. von dem jeweiligen Handgriff bzw. Betätigungshebel an dem sie befestigt sind, abnehmbar.

Ausgehend von diesen Blattfederkonstruktionen wird beim Gegenstand der DE 20 2004 017 974 U1 eine Rückstellfeder in Form einer Schraubendruckfeder (Axialdruckfeder) eingesetzt, welche mittels eines die Rückstellfeder axial durchragenden Führungsstiftes wenigstens teilweise in einer Führungsnut der Gleitfläche des Stanzschiebers angeordnet und geführt ist. Als Stützlager für das rückwärtige distale Federende der Rückstellfeder ist eine mit dem Stanzschieber fest verbundene distale Anschlagfläche vorgesehen. Des Weiteren liegt die Rückstellfeder mit ihrem vorderen proximalen Ende bei abgenommenem Stanzschieber an einer proximalen Anschlagfläche des Stanzschiebers an. Bei arbeitsgerecht montiertem Stanzschieber liegt die Rückstellfeder an einer proximalen Anschlagfläche des Führungsschaftes selbst an.

Das heißt, dass bei dieser Konstruktion die Rückstellfeder integraler Bestandteil des Führungsschaftes und des Stanzschiebers ist, so dass diese nach außen hin nicht erkennbar ist. Diese Rückstellfeder gelangt beim Abnehmen des Stanzschiebers vom Führungsschaft automatisch außer Wirkverbindung, so dass insbesondere die Montage und Demontage des Stanzschiebers auf dem Führungsschaft erheblich vereinfacht ist.

In neuerer Zeit ist es jedoch gewünscht, dass nach dem Lösen der Führungselemente der Stanzschieber zwar vom aus dem Führungsschaft, dem Verbindungsbereich und dem Handgriff bestehenden Grundkörper getrennt werden kann, der Stanzschiebers und der Grundkörper jedoch in irgendeiner Art und Weise noch miteinander in Verbindung bleiben, so dass diese beiden Teile nicht vollständig voneinander getrennt werden können, und beispielsweise zusammen gereinigt werden können. Da insbesondere mehrere Knochenstanzen in einem gemeinsamen Reinigungsprozess gereinigt werden sollen, hat eine solche unverlierbare "Sicherung" den Vorteil, dass die zusammengehörenden Bauteile einer Knochenstanze stets wieder korrekt zusammengefügt werden können.

Diesbezüglich ist aus der DE 10 2009 056 099 B4 eine als chirurgisches Instrument bezeichnete Knochenstanze bekannt, bei welcher eine derartige Funktionsweise nach dem Trennen des Stanzschiebers vom Führungsschaft bzw. vom Grundkörper vorgesehen ist. Dies wird bei diesem Gegenstand dadurch erreicht, dass der Stanzschieber in der vom Führungsschaft getrennten Position am Führungsschaft schwenkbar angeordnet ist. Der Führungsschaft weist hierzu an seinem proximalen Ende ein Langloch auf, in dessen Bereich der Stanzschieber zwei voneinander beabstandete seitlich und nach unten bis über das Langloch hinausragende Wangen aufweist, welche mit einem Lagerstift in Verbindung stehen der wiederum das Langloch durchgreift. Dieser Lagerstift ist dabei als eine Art Schraube ausgebildet und steht mit den beiden Wangen feststehend in Verbindung. Dabei ist vorgesehen, dass der Führungsschaft bzw. der Grundkörper in dem an das Langloch angrenzenden Bereich eine verringerte Breite aufweist, so dass die beiden Wangen seitlich nicht über den Führungsschaft bzw. den Verbindungsbereich des Führungsschaftes zum Handgriff hinausstehen.

Bei dieser Konstruktion sind die "Sicherungsmittel" in Form des Langloches sowie der beiden Wangen mit dem Sicherungsstift von außen frei zugänglich, so dass diese verschmutzen können. Des Weiteren ist hier eine spezielle Ausgestaltung des Verbindungsbereiches zwischen dem Führungsschaft und dem Handgriff vorgesehen, da dieses Langloch sich doch über einen erheblichen Stellweg in Längsrichtung des Führungsschaftes distal nach hinten erstrecken muss.

Demgemäß liegt der Erfindung die Aufgabe zugrunde, insbesondere ausgehend von der DE 20 2004 017 974 U1, eine Knochenstanze mit einem Führungsschaft und einem Stanzschieber zu schaffen, bei welcher nach der Trennung der Führungselemente zwischen dem Führungsschaft und dem Stanzschieber der Stanzschieber mit seiner Gleitfläche von der Führungsfläche des Führungsschaftes zwar abgenommen werden kann, jedoch weiterhin mit dem Führungsschaft bzw. dem Verbindungsbereich des Führungsschaftes zum Handgriff insbesondere schwenkbeweglich in Verbindung bleibt.

Die Aufgabe wird erfindungsgemäß dadurch gelöst, dass der Stanzschieber in seinem distalen Endbereich ein Sicherungselement aufweist, welches im montierten Zustand des Stanzschiebers auf dem Führungsschaft zwischen die beiden Seitenwände hineinragt und dass das Sicherungselement zwei seitlich vorstehende Formschlusselemente aufweist und dass jede Seitenwand mit einer inneren in Längsrichtung des Führungsschaftes verlaufenden, distal begrenzten Längsnut versehen ist, in welche jeweils ein Formschlusselement längs verschiebbar einsetzbar.

Durch die erfindungsgemäße Ausgestaltung wird eine Knochenstanze zur Verfügung gestellt, bei welcher nach dem Trennen der Führungselemente zwischen dem Führungsschaft und dem Stanzschieber der Stanzschieber weiterhin zumindest schwenkbeweglich mit dem Führungsschaft bzw. dem Verbindungsbereich zwischen Führungsschaft und Handgriff oder Handgriff in Verbindung bleibt. Dabei sind das Sicherungselement des Stanzschiebers und die zugehörigen, inneren Längsnuten im Bereich der Seitenwände des Grundkörpers derart angeordnet, dass diese im normalen Betriebszustand der erfindungsgemäßen Knochenstanze nach außen nicht zugänglich sind. Dementsprechend weist die erfindungsgemäße Knochenstanze nach außen hin im normal montierten Betriebszustand dasselbe Erscheinungsbild auf wie die nach dem nächstliegenden Stand der Technik der DE 20 2004 017 974 U1 ausgestaltete Knochenstanze.

Weitere vorteilhafte Ausgestaltungen der Erfindung sind den weiteren Unteransprüchen zu entnehmen.

So kann gemäß Anspruch 2 vorgesehen sein, dass das Sicherungselement einen sich zum Stanzschieber hin erstreckenden Lagerabschnitt aufweist, mit welchem das Sicherungselement lösbar mit dem Stanzschieber in Verbindung steht. Zur lösbaren Verbindung kann hier beispielsweise eine Schraubverbindung vorgesehen sein. Diese Schraubverbindung ist insbesondere bei vom Grundkörper gelöstem Stanzschieber von außen zugänglich, so dass der Stanzschieber bei Bedarf nach dem Lösen der Schraubverbindung gänzlich vom Sicherungselement und damit vom Grundkörper getrennt werden kann.

Die Ausgestaltung nach Anspruch 3 orientiert sich als Ausgangspunkt am Gegenstand der DE 20 2004 017 974 U1. Die Merkmalskombination nach Anspruch 3 kann als alternative Ausgestaltung zu einer Schraubverbindung zwischen dem Stanzschieber und dem Sicherungselement vorgesehen sein. Danach ist als Rückstellfeder eine Axialdruckfeder vorgesehen, welche auf einem Führungsstift angeordnet ist und im montierten Zustand teilweise in einer unteren Aufnahmenut des Stanzschiebers angeordnet ist, wobei der Führungsstift im montierten Zustand proximal in eine Aufnahmebohrung des Stanzschiebers und distal in eine zweite Bohrung des Stanzschiebers abnehmbar eingesetzt ist. Des Weiteren stützt sich die Axialdruckfeder bei vom Führungsschaft abgenommenem Stanzschieber proximal an einem ersten Anschlag des Stanzschiebers axial ab, wobei sich die Axialdruckfeder an einem distalen zweiten Anschlag des Stanzschiebers axial abstützt. Die Axialdruckfeder wird folglich zwischen diesen beiden Anschlägen aufgenommen, wobei die Anschläge zumindest teilweise von der Aufnahmenut gebildet sein. Das Sicherungselement wird bei dieser alternativen Ausgestaltung mit seinem Lagerabschnitt zwischen dem distalen Ende der Axialdruckfeder und dem zweiten Anschlag aufgenommen. Durch diese Ausgestaltung wird die Montage und Demontage des Sicherungselementes erleichtert, da keine Schraubverbindung vorzusehen ist, sondern das Sicherungselement mit seinem Lagerabschnitt einfach "klemmend" zwischen der Axialdruckfeder und dem distalen Anschlag aufgenommen ist. Beim Einsetzen des Führungsstiftes zusammen mit der Axialdruckfeder, ähnlich wie bei einer Ausführungsvariante des Gegenstandes der DE 20 2004 017 974 U1, wird aufgrund der Anordnung des Sicherungselementes mit seinem Lagerabschnitt, der dann ebenfalls auf dem Führungsstift im distalen Endbereich der Axialdruckfeder angeordnet ist, das Sicherungselement zusammen mit dem Führungsstift und der Axialdruckfeder entsprechend gleichzeitig am Stanzschieber im Bereich der Aufnahmenut montiert.

Zur weiteren Vereinfachung der Montage kann gemäß Anspruch 4 vorgesehen sein, dass der Führungsstift in seinem distalen Endbereich einen radial vorstehenden Anschlagbund aufweist, über welchen sich die Axialdruckfeder mittelbar am zweiten, distalen Anschlag des Standschiebers axial abstützt und dass das Sicherungselement mit seinem Lagerabschnitt zwischen dem distalen Ende der Axialdruckfeder und dem Anschlagbund des Führungsstiftes auf dem Führungsstift aufgenommen ist. Durch diese Ausgestaltung wird das Sicherungselement mit seinem Lagerabschnitt zwischen dem distalen Ende der Axialdruckfeder und dem Anschlagbund des Führungsstiftes schon vor der Montage am Führungsstift fixieret, so dass deren gemeinsame Handhabung entsprechend einfacher erfolgen kann.

Weiter kann gemäß Anspruch 5 vorgesehen sein, dass der erste, proximale Anschlag Teil eines ersten T-förmig ausgebildeten Kupplungselementes des Stanzschieber ist, welches zur Führungsfläche des Führungsschaftes hin über die Gleitfläche des Stanzschiebers hinaussteht und im montierten Zustand mit einer T-förmig ausgebildeten Aufnahmenut längs verschiebbar in Eingriff steht und dass der zweite, distale Anschlag Teil eines zweiten T-förmig ausgebildeten Kupplungselementes des Stanzschiebers ist, welches zur Führungsfläche des Führungsschaftes hin über die Gleitfläche des Stanzschiebers hinaussteht und im montierten Zustand mit inneren, seitlich in den Seitenwänden angeordneten Längsnuten längs verschiebbar in Eingriff steht und dass die beiden Anschläge gleichzeitig auch Teil der Aufnahmenut des Stanzschiebers für die Axialdruckfeder sind. Auf Grund dieser Ausgestaltung ist der Führungsstift zusammen mit dem Sicherungselement und der Axialdruckfeder in einfacher Weise zwischen die beiden Anschläge einbringbar. Die beiden Kupplungselemente können zur Aufnahme des Führungsstiftes beispielsweise zum Führungsschaft hin "offene" Rastgabeln aufweisen, in welche der Führungsstift rastend und axial unverschiebbar einsetzbar ist.

Durch die Ausgestaltung nach Anspruch 6 ist der Führungsstift zusammen mit dem auf dem Führungsstift aufgesetzten Sicherungselement sowie der auf den Führungsstift aufgeschobenen Axialdruckfeder äußerst einfach montierbar bzw. demontierbar. Danach kann vorgesehen sein, dass der Stanzschieber im Bereich seines ersten, proximalen Kupplungselementes mit einer Aufnahmebohrung versehen ist, in welche der Führungsstift mit einem proximalen, radial verjüngt ausgebildeten Steckzapfen einsteckbar ist und dass der Stanzschieber im Bereich seines zweiten, distalen Kupplungselementes eine Durchgangsbohrung aufweist und dass der Führungsstift in distaler, axialer Verlängerung zu seinem Anschlagbund einen radial verjüngt ausgebildeten Rastzapfen aufweist, mit welchem der Führungsstift durch eine Axialverstellung des Führungsstiftes in der Aufnahmebohrung entgegen der Federkraft der auf den Führungsstift aufgesetzten Axialdruckfeder mit der Durchgangsbohrung im Bereich des zweiten Kupplungselementes formschlüssig in Eingriff bringbar ist. Durch diese Ausgestaltung ist der Führungsstift an den beiden Kupplungselementen im Bereich der beiden Anschläge unverlierbar gehalten und kann sich auch während des Betriebs und auch bei vom Führungsschaft "abgehobenem" Stanzschieber nicht selbständig lösen.

Anhand der Zeichnung wird nachfolgend die Erfindung beispielhaft näher erläutert. Es zeigt:
- Fig. 1: eine perspektivische Darstellung einer erfindungsgemäßen Knochenstanze im normalen, montierten Betriebszustand;
- Fig. 2: eine perspektivische Teilansicht des aus dem Führungsschaft, dem Verbindungsbereich und dem Handgriff bestehenden Grundkörper der Knochenstanze aus Fig. 1 sowie eine perspektivische Unteransicht des Stanzschiebers jeweils in verkürzter Darstellung;
- Fig. 3: ein Sicherungselement, eine als Schraubendruckfeder ausgebildete Rückstellfeder sowie einen Führungsstift in perspektivischer Explosionsdarstellung;
- Fig. 4: die Bauteile aus Fig. 3 im montierten Zustand in perspektivischer Darstellung;
- Fig. 5: einen Vertikalschnitt durch den distalen Endbereich des Stanzschiebers zusammen mit den montierten Bauteilen gemäß Fig. 4 unmittelbar vor der Montage am Stanzschieber in ihrem am Stanzschieber angesetzten Zustand;
- Fig. 6: eine perspektivische Darstellung des distalen Endbereiches des Stanzschiebers in perspektivischer Unteransicht mit den Bauteilen aus Fig. 4 in ihrem am Stanzschieber montierten Zustand;
- Fig. 7: einen Vertikalschnitt durch den Verbindungsbereich zwischen Führungsschaft und Handgriff mit eingesetztem Stellhebel des Betätigungshebels sowie eingesetztem Sicherungselement;
- Fig. 8: die Darstellung aus Fig. 7 zusammen mit dem im distalen Endbereich des Verbindungsbereich angeordneten Sicherungselement aus Fig. 3 sowie dem mit dem Sicherungselement in Verbindung stehenden Führungsstift sowie der auf den Führungsstift aufgesetzten Axialdruckfeder;
- Fig. 9: den Vertikalschnitt aus Fig. 8 mit am Führungssstift montiertem Stanzschieber;
- Fig. 10: die Darstellung aus Fig. 9 mit auf dem Führungsstift aufliegendem Stanzschieber in seiner distal zurückgezogenen Position;
- Fig. 11: die Darstellung aus Fig. 10 mit dem Stanzschieber sowie dem Betätigungshebel mit seinem Stellhebel in ihrer normalen Betriebsposition im Vertikalschnitt.

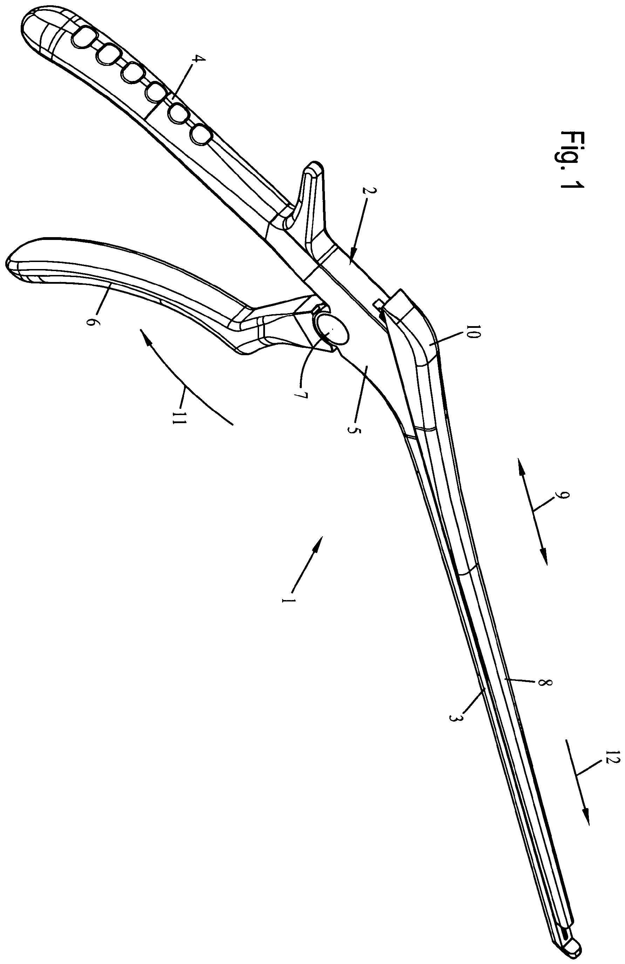

Fig. 1 zeigt eine perspektivische Darstellung einer Knochenstanze 1. Diese Knochenstanze 1 weist einen Grundkörper 2 auf, der im Wesentlichen aus einem Führungsschaft 3, einem Handgriff 4 sowie einem zwischen dem Handgriff 4 und dem Führungsschaft 3 vorgesehenen Verbindungsbereich 5 besteht. In diesem Verbindungsbereich 5 ist ein Betätigungshebel 6 schwenkbar gelagert, wozu ein entsprechender Lagerzapfen 7 vorgesehen ist. Die spezielle Ausgestaltung, insbesondere im Bezug auf die Ausgestaltung des Lagerzapfens 7 und der speziellen Lagerung des Betätigungshebels 6, ist der DE 20 2004 017 974 U1 entnehmbar. Das heißt, dass die Lagerung bei der dargestellten Ausführungsvariante der Knochenstanze 1 des Betätigungshebels 6 im Verbindungsbereich 5 vorzugsweise identisch ausgebildet ist wie beim Gegenstand der DE 20 2004 017 974 U1.

Weiter ist aus Fig. 1 erkennbar, dass auf dem Führungsschaft 3 ein Stanzschieber 8 angeordnet ist, welcher in Längsrichtung des Doppelpfeiles 9 entlang des Führungsschaftes 3 verstellbar ausgebildet ist. Mit seinem distalen Endbereich 10 steht der Stanzschieber 8 mit einem aus Fig. 1 nicht erkennbaren Stellhebel des Betätigungshebels 6 in Wirkverbindung, so dass bei Betätigen des Betätigungshebels 6 in Richtung des Pfeiles 11 der Stanzschieber 8 in Richtung des Pfeiles 12 verstellt wird, wie dies aus dem Stand der Technik bekannt ist.

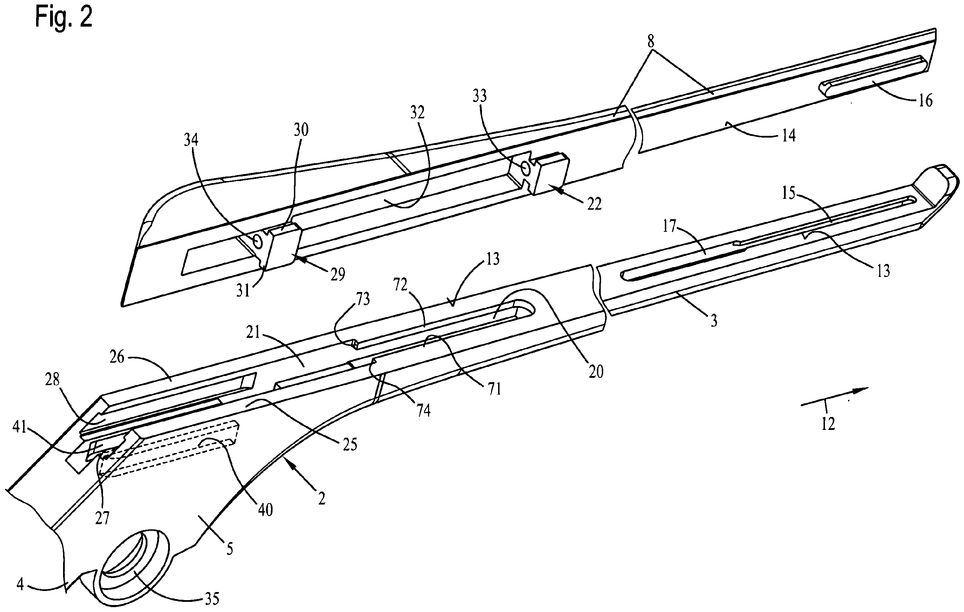

Fig. 2 zeigt eine perspektivische Explosionsdarstellung des Grundkörpers 2 mit seinem Führungsschaft 3, dem Verbindungsbereich 5 und einem Teil des Handgriffes 4 in perspektivischer Oberansicht sowie eine perspektivische Unteransicht des Stanzschiebers 8, wobei sowohl der Führungsschaft 3 als auch der Stanzschieber 8 in ihrer Länge verkürzt dargestellt sind.

Es ist erkennbar, dass der Führungsschaft 3 "oberseitig" dem schräg nach unten am Führungsschaft 3 angeordneten Handgriff gegenüber liegend eine ebene Führungsfläche 13 aufweist, auf welcher der Stanzschieber 8 mit einer unteren Gleitfläche 14 im in Fig. 1 dargestellten, montierten Betriebszustand längs verschiebbar aufliegt. Im Bereich der Führungsfläche 13 weist der Führungsschaft 3 in seinem proximalen Endbereich eine T-förmig ausgebildete Aufnahmenut 15 auf, welche mit einer T-förmig ausgebildeten, über die Gleitfläche 14 nach unten zum Führungsschaft 3 hin vorstehende Führungsleiste 16 des Stanzschiebers 8 in Wirkverbindung bringbar ist. Um die Führungsleiste 16 mit der Aufnahmenut 15 in Eingriff bringen zu können, schließt sich an die Aufnahmenut 15 distal eine seitlich erweiterte Einführungsnut 17 an, in welche die Führungsleiste 16 passend einsetzbar ist. Nach dem Einsetzten der Führungsleist 16 in die Einführungsnut 17 und dem Verstellen des Stanzschiebers 8 in Richtung des Pfeiles 12 gelangt die T-förmige Führungsleiste 16 längs verschiebbar mit der T-förmig ausgebildeten Aufnahmenut 15 des Führungsschaftes 3 in Eingriff.

Zum Verbindungsbereich 5 hin weist der Führungsschaft 3 im Bereich seiner Führungsfläche 13 eine zweite T-förmig ausgebildete Aufnahmenut 20 auf, an welche sich distal ebenfalls eine breiter ausgebildete Einführungsnut 21 anschließt. Im entsprechenden axialen Bereich ist an der unteren Gleitfläche 14 des Stanzschiebers 8 ein T-förmig ausgebildetes erstes Kupplungselement 22 vorgesehen, welches im normalen Betriebszustand entsprechend mit der Aufnahmenut 20 längs verschiebbar in Eingriff steht. Um dieses Kupplungselement 22 mit der Einführungsnut 21 in Eingriff bringen zu können, wird der Stanzschieber 8 in einer entgegen des Pfeiles 12 versetzt zum Führungsschaft 3 befindlichen Position auf den Führungsschaft 3 aufgesetzt, so dass das Kupplungselement 22 in die Einführungsnut 21 hineinragt. Durch anschließendes Verstellen des Stanzschiebers 8 in Richtung des Pfeiles 12 gelangt das T-förmige Kupplungselement 22 somit mit der T-förmigen Aufnahmenut 20 in Eingriff, wie dies für die Führungsleiste 16 und die Aufnahmenut 15 ebenfalls der Fall ist.

Bei der dargestellten Ausführungsvariante bildet der Grundkörper 2 im Verbindungsbereich 5 zwischen dem Führungsschaft 3 und dem teilweise erkennbaren Handgriff 4 zwei Seitenwände 25, 26 zwischen welchen der Betätigungshebel aus Fig. 1 mit einem entsprechenden Stellhebel (in der Zeichnung nicht dargestellt) schwenkbeweglich aufgenommen wird. Zur oberen Führungsfläche 13 hin weisen die beiden Seitenwände 25 und 26 jeweils eine distal offene Längsnut 27 bzw. 28 auf, mit welcher ein zweites T-förmiges Kupplungselement 29 in Eingriff bringbar ist. Dabei wird der Stanzschieber 8 in einer entgegen des Pfeiles 12 versetzt zum Führungsschaft 3 angeordneten Montageposition auf den Führungsschaft 3 aufgesetzt und in Richtung des Pfeiles 12 verstellt. Dabei gelangt das T-förmige Kupplungselement 29 mit seinen beiden seitlich vorstehenden Führungsstegen 30 bzw. 31 mit den Längsnuten 27 und 28 in Eingriff.

Des Weiteren ist aus Fig. 2 erkennbar, dass die beiden Kupplungselemente 22 und 29 in axialer Richtung des Pfeiles 12 einen Abstand voneinander aufweisen und dass der Stanzschieber 8 unterseitig eine Aufnahmenut 32 aufweist, welche sich beim vorliegenden Ausführungsbeispiel zwischen den beiden Kupplungselementen 22 und 29 erstreckt, deren Funktion später noch näher erläutert wird. Des Weiteren weist das Kupplungselement 22 eine Aufnahmebohrung 33 auf, in welche ein Führungsstift einsetzbar ist. Das Kupplungselement 29 weist eine Bohrung 34 auf, w4elche beim vorliegenden Ausführungsbeispiel als Durchgangsbohrung 34 ausgebildet ist und welche ebenfalls mit dem oben erwähnten Führungsstift koppelbar ist. Dies wird später noch näher erläutert.

Des Weiteren ist aus Fig. 2 erkennbar, dass die beiden Seitenwände 25 und 26 des Grundkörpers 2 bzw. des Verbindungsbereiches 5 vertikal "unterhalb" der beiden Längsnuten 27 und 28 zur Lagerbohrung 35 des Verbindungsbereiches 5 hin, zwei weitere Längsnuten 40 und 41 aufweisen, welche distal zum Handgriff 4 hin begrenzt sind und eine Art distalen Anschlag bilden. Diese beiden Längsnuten 40 und 41 münden in die Einführungsnut 21, so dass in diese Längsnuten 40 und 41 ein entsprechendes Sicherungselement über die Einführungsnut 21 einsetzbar ist.

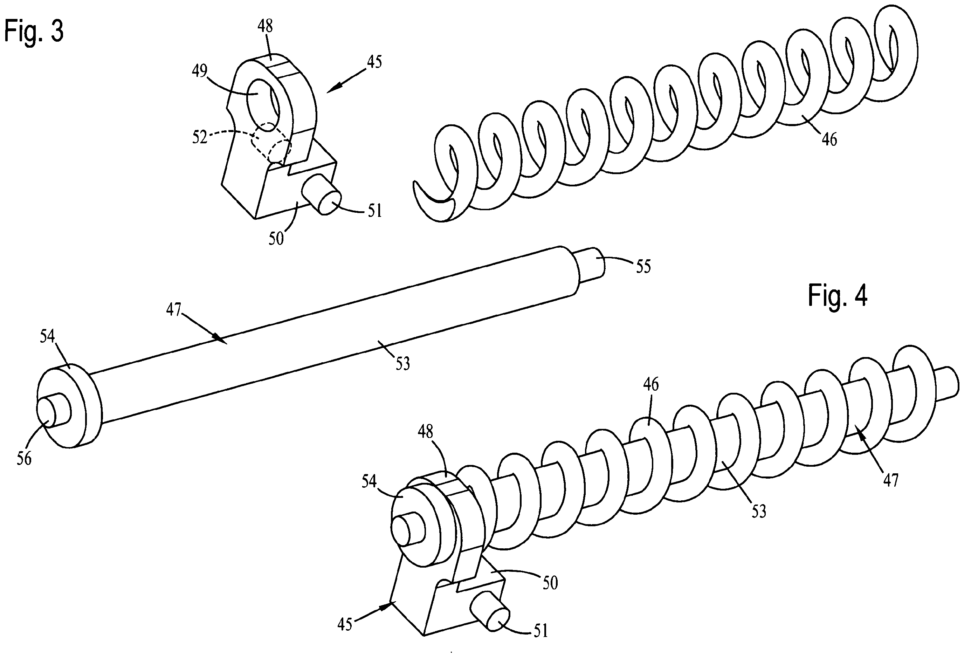

Fig. 3 zeigt eine perspektivische Darstellung eines solchen Sicherungselementes 45 zusammen mit einer Axialdruckfeder 46 sowie einem Führungsstift 47 in perspektivischer Explosionsdarstellung. Das Sicherungselement 45 bildet einen vertikal nach "oben" ausgerichteten plattenförmigen Lagerabschnitt 48, welcher eine Lagerbohrung 49 aufweist. Am Lagerabschnitt 48 ist unterseitig ein Führungssteg 50 vorgesehen, welcher beim dargestellten Ausführungsbeispiel quer zum Lagerabschnitt 48 verläuft und sich ausgehend vom Lagerabschnitt proximal nach vorne erstreckt. Der Führungssteg 50 weist zwei seitlich vorstehende zapfenartig ausgebildete Formschlusselemente 51 und 52 auf. Das Sicherungselement 45 ist zwischen die beiden Seitenwände 25, 26 des Grundkörpers 2 passend einsetzbar, wie später noch näher erläutert wird.

Der Führungsstift 47 bildet einen zentralen, zylindrischen Aufnahmeabschnitt 53, welcher beim vorliegenden Ausführungsbeispiel in seinem distalen Endbereich durch einen umlaufenden, radial erweiterten Anschlagbund 54 begrenzt ist. In seinem, diesem Anschlagbund 54 gegenüber liegenden, proximalen Endbereich weist der Aufnahmeabschnitt 53 einen Steckzapfen 55 auf, welcher beim vorliegenden Ausführungsbeispiel radial verjüngt ausgebildet ist. Diesem Steckzapfen 55 axial gegenüberliegend, in axialer, distaler Verlängerung zum Anschlagbund 54 bildet der Führungsstift 47 einen zylindrischen Rastzapfen 56, welcher ebenfalls radial verjüngt ausgebildet ist.

Wie aus Fig. 4 ersichtlich ist, ist das Sicherungselement 45 mit der Lagerbohrung 49 seines Lagerabschnittes 48 auf den Aufnahmeabschnitt 53 des Führungsstiftes 47 aufschiebbar und kann entsprechend am Anschlagbund 54 des Führungsstiftes 47 zur Anlage gebracht werden. Dabei weist der Führungssteg 50 des Sicherungselementes 45 mit seinen beiden Formschlusselementen 51 und 52 (in Fig. 4 nur das Formschlusselement 51 sichtbar) einen Abstand zum Aufnahmeabschnitt 53 des Führungsschaftes 47 auf. Nachdem das Sicherungselement 45 mit seinem Lagerabschnitt 48 auf den Aufnahmeabschnitt 53 des Führungsstiftes 47 aufgeschoben ist, kann die Axialdruckfeder 46 ebenfalls auf den Aufnahmeabschnitt 53 des Führungsstiftes 47 aufgesteckt werden. Diesen montierten Zustand zeigt die perspektivische Darstellung der Fig. 4. In diesem fertig montierten Zustand ist der Führungsstift 47 zusammen mit der Axialdruckfeder 46 sowie dem Sicherungselement 45 unterseitig am Stanzschieber 8 zwischen den beiden Kupplungselementen 22 und 29 einsetzbar.

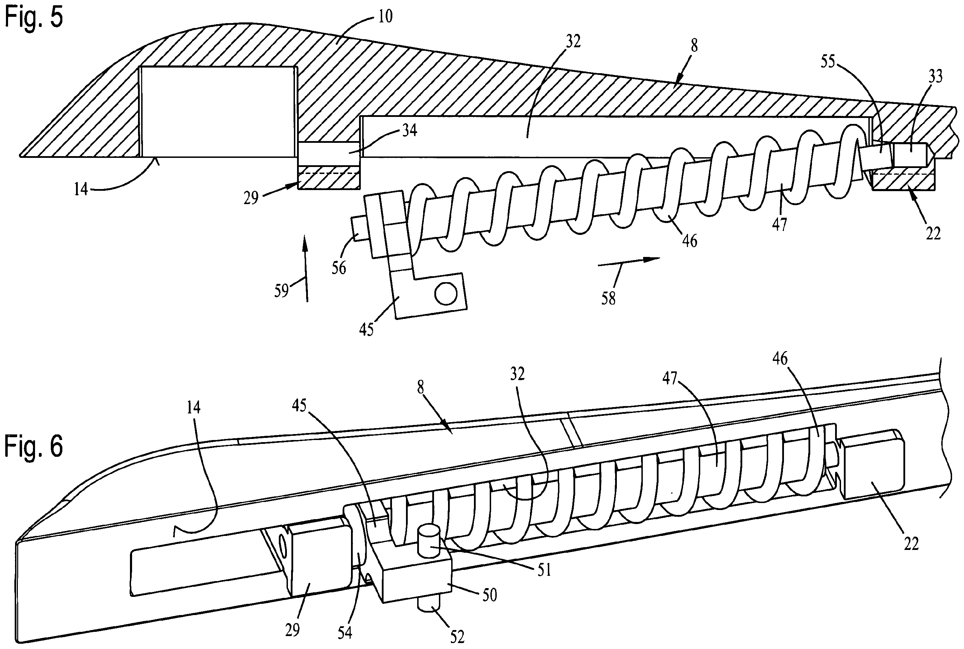

Hierzu zeigt Fig. 5 einen Vertikalschnitt des Stanzschiebers 8 in seinem distalen Endbereich 10. In diesem distalen Endbereich 10 sind die beiden Kupplungselemente 22 und 29 angeordnet, welche vertikal nach unten über die untere Gleitfläche 14 des Stanzschiebers 8 vorstehen. Des Weiteren ist aus Fig. 5 einerseits die Aufnahmebohrung 33 sowie die als Durchgangsbohrung ausgebildete Bohrung 34 des Kupplungselementes 22 bzw. 29 erkennbar. Zum Einsetzen des Führungsstiftes 47 zusammen mit der aufgeschobenen Axialdruckfeder 46 sowie dem montierten Sicherungselement 45 wird der Führungsstift 47 zunächst mit seinem proximalen Steckzapfen 55 mit der Aufnahmebohrung 33 in Eingriff gebracht, wie dies aus Fig. 5 ersichtlich ist. Anschließend wird der Führungsstift 47 in Richtung des Pfeiles 58 unter Komprimierung der Axialdruckfeder 46 weiter in die Aufnahmebohrung 33 eingeschoben, bis dessen Rastzapfen 56 in Richtung des Pfeiles 59 in die Aufnahmenut 32 des distalen Kupplungselementes 29 verstellbar ist. Die Aufnahmebohrung 33 kann zum Ansetzen und Einschieben des Steckzapfens 55 leicht konisch ausgebildet sein, wie dies in Fig. 5 angedeutet ist. Sobald der Rastzapfen 56 auf die Durchgangsbohrung 34 des Kupplungselementes 29 ausgerichtet ist und der Führungsstift 47 freigegeben ist, wird aufgrund der Federkraft der Axialdruckfeder 46 der Führungsstift 47 wiederum entgegen des Pfeiles 58 verstellt, so dass der Rastzapfen 56 formschlüssig in die Durchgangsbohrung 34 des Kupplungselementes 29 eingreift. Damit ist der Führungsstift 47 zusammen mit der Axialdruckfeder 46 sowie dem Sicherungselement 45 im Bereich zwischen den beiden Kupplungselementen 22 und 29 am Stanzschieber 8 montiert. In diesem montierten Zustand wird die Axialdruckfeder 46 teilweise in der sich zwischen den beiden Kupplungselementen 22 und 29 erstreckenden Aufnahmenut 32 aufgenommen. Diese Aufnahmenut 32 bildet zusammen mit den beiden Kupplungselementen 22 und 29 jeweils einen Anschlag, an welchen die Axialdruckfeder 46 axial sowohl proximal aus auch distal abstützt.

Diese fertig montierte "Betriebsposition" zeigt die perspektivische Unteransicht der Fig. 6.

Es ist erkennbar, dass die Axialdruckfeder 46 zusammen mit dem Führungsstift 47 zwischen den beiden Kupplungselementen 22 und 29 aufgenommen wird und teilweise in die Aufnahmenut 32 des Stanzschiebers 8 hineinragt. Das Sicherungselement 45 wird zwischen dem distalen Ende der Axialdruckfeder und dem radial erweiterten Anschlagbund 54 klemmend aufgenommen und steht nach unten über die untere Gleitfläche 14 des Stanzschiebers 8 hinaus, wobei in dieser Position im normalen Betriebszustand die beiden Formschlusselemente 51 und 52 mit den beiden zu Fig. 2 beschriebenen Längsnuten 40 und 41 des Verbindungsbereiches 5 des Grundkörpers 2 der Knochenstanze 1 längs verschiebbar in Eingriff stehen.

Um das Sicherungselement 45 mit seinen beiden Formschlusselementen 51 und 52 mit den beiden inneren Längsnuten 40 und 41 der beiden Seitenwände 25 und 26 des Grundkörpers 2 in Eingriff bringen zu können, wird das Sicherungselement 45 als "Einzelteil" zunächst ohne den Führungsstift 47 und ohne die Axialdruckfeder 46 in die aus Fig. 2 erkennbare Einführungsnut 21 eingesetzt. Dabei gelangen die beiden Formschlusselemente 51 und 52 in den vertikalen Bereich der beiden proximal "offenen", in die Einführungsnut 21 mündenden Längsnuten 40 und 41 und können somit entgegen des Pfeiles 12 durch Verschieben in distaler Richtung mit diesen beiden Längsnuten 40 und 41 in Eingriff gebracht werden.

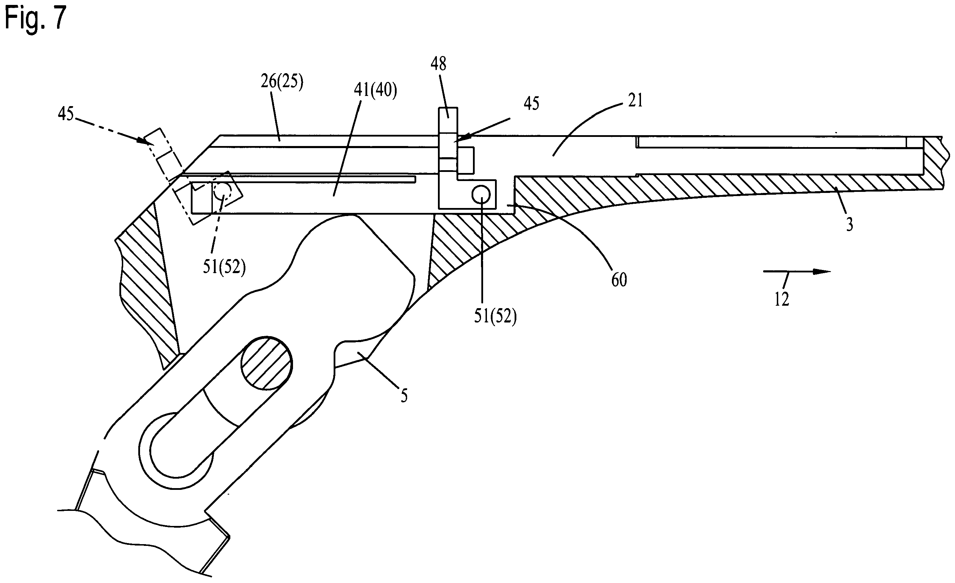

Hierzu zeigt Fig. 7 das Sicherungselement 45 mit seinem Lagerschaft 48 sowie seinem Führungssteg 50 in durchgezogenen Linien in dem in die Einführungsnut 21 eingesetzten Zustand. Die Einführungsnut 21 ist beim vorliegenden Ausführungsbeispiel abgesetzt ausgebildet und weist einen nach innen bzw. nach unten abgesetzten, vertieften Aufnahmeabschnitt 60 auf, in welchen die beiden Längsnuten 40 und 41 müden und in welchen das Sicherungselement 45 mit seinem Führungsstegen 50 eingesetzt ist. Durch Verschieben des Sicherungselementes 45 entgegen des Pfeiles 12 gelangt der Führungssteg 50 mit seinen beiden Formschlusselementen 51, 52 mit den Längsnuten 40 und 41 in Eingriff, wobei in Fig. 7 lediglich die Längsnut 41 der "hinteren" Seitenwand 26 erkennbar ist.

Nachdem das Sicherungselement 45 in die in Fig. 7 in Phantomlinien dargestellte "hintere" distale Endposition gebracht wurde, kann das Sicherungselement 45 durch Verschwenken um ihre Formschlusselemente 51, 52 in eine Winkelposition relativ zum Verbindungabschnitt 5 gebracht werden, in welcher das Sicherungselement 45 mit dem Führungsstift 47 sowie der Axialdruckfeder 46 in Verbindung gebracht werden kann, wie dies zu Fig. 4 dargestellt und beschrieben wurde. Da das Sicherungselement insbesondere mit seinem Führungssteg passend zwischen die beiden Seitenwände 25 und 26 eingesetzt ist, kann das Sicherungselement nicht um eine im Lagerabschnitt 48 liegende Achse gedreht werden.

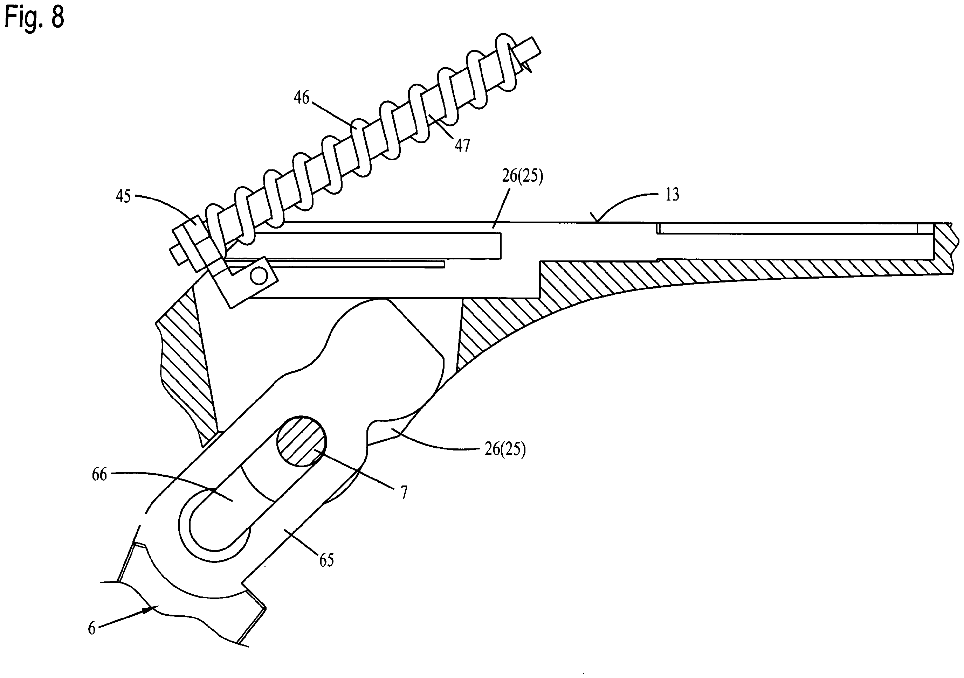

Dabei ist der Abstand der beiden Seitenwände 25 und 26 derart bemessen, dass insbesondere die Axialdruckfeder 46 zwischen diesen Platz findet. Damit ist sichergestellt, dass der Führungsstift 47 und die Axialdruckfeder 46 auch mit dem Sicherungselement 45 in Eingriff gebracht werden können, wie dies aus der Schnittdarstellung der Fig. 8 erkennbar ist.

Des Weiteren ist aus Fig. 8 ebenfalls erkennbar, dass der Betätigungshebel 6 einen Stellhebel 65 aufweist, welcher im Bereich der beiden Seitenwände 25 und 26 aufgenommen wird, wobei in Fig. 8 lediglich die "hintere" Seitenwand 26 erkennbar ist. Im Bereich dieser beiden Seitenwände 25 und 26 ist der zu Fig. 1 erwähnte Lagerzapfen 7 erkennbar, an welchem der Betätigungshebel 6 mit seinem Stellhebel 65 über ein Langloch 66 schwenkbar und bei Bedarf radial zum Lagerzapfen 7 verstellbar aufgenommen ist.

Die genaue Ausgestaltung der Lagerung sowie des Lagerzapfens 7 und die Ausgestaltung des Stellhebels 65 ist aus der DE 20 2004 017 974 U1 insbesondere aus der Beschreibung zu den Zeichnungsfiguren 20 bis 23 entnehmbar. Der prinzipielle, funktionelle und konstruktive Aufbau dieser Lagerung ist ebenfalls beim Erfindungsgegenstand vorgesehen, so dass auf diese Beschreibung der genannten Druckschrift voll umfänglich Bezug genommen wird und diese auch Inhalt der vorliegenden Anmeldung ist.

An dieser Stelle sei allerdings angemerkt, dass der Betätigungshebel auch anderweitig am Grundkörper drehbar und verstellbar gelagert sein kann, wie dies aus dem Stand der Technik ebenfalls bekannt ist. Es ist lediglich zu gewährleisten, dass der Stellhebel durch entsprechende Manipulation mit dem Stanzschieber außer Eingriff gebracht werden kann, um den Stanzschieber vom Führungsschaft bzw. Grundkörper trennen zu können.

Wie aus Fig. 8 weiter ersichtlich ist, verläuft der Führungsstift 47 in seinem mit dem Sicherungselement 45 in Eingriff stehenden Zustand zusammen mit der Axialdruckfeder 46 zunächst schräg zur oberen Führungsfläche 13 des Führungsschaftes 3. In dieser Winkelposition kann nunmehr der Stanzschieber 8 aus Fig. 2, wie zu den Fig. 5 und 6 beschrieben, mit dem Führungsstift 47 in Eingriff gebracht werden. Das heißt letztendlich, dass der Stanzschieber 8 in einfacher Weise am Führungsstift 47 eingehängt bzw. an diesem "eingerastet" wird.

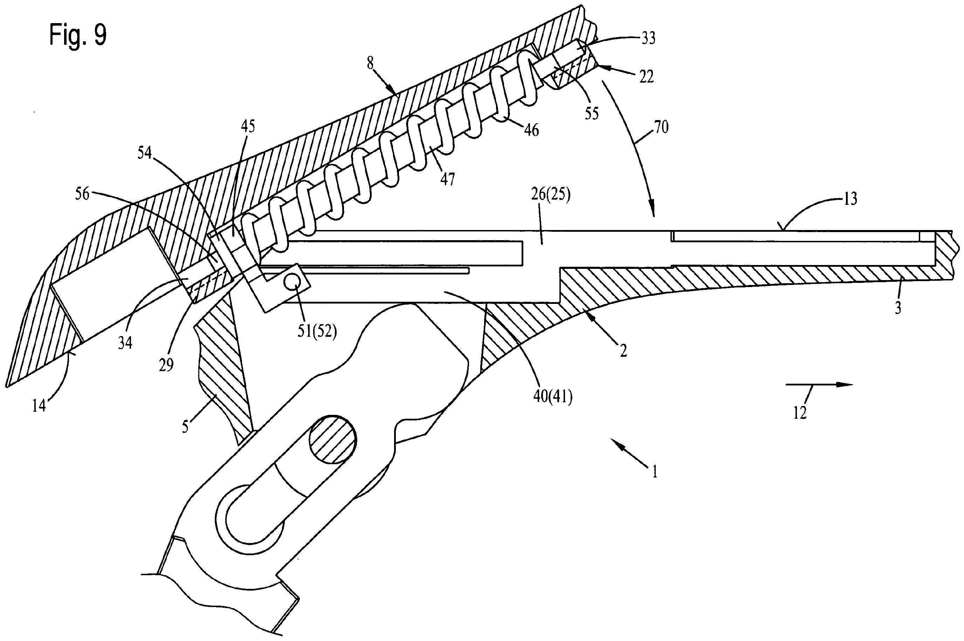

Diesen fertig montierten Zustand zeigt die Schnittdarstellung der Fig. 9.

Wie aus der Schnittdarstellung der Fig. 9 erkennbar ist, ist der Führungsstift 47 mit seinem Steckzapfen 55 in die Aufnahmebohrung 33 des Kupplungselementes 22 eingesteckt. Auf dem Aufnahmeabschnitt 53 des Führungsstift 47 ist die Axialdruckfeder 46 aufgenommen. Diese stützt sich proximal am Kupplungselement 22 ab und fixiert das Sicherungselement 45 am distalen Anschlagbund 54 des Führungsstiftes 47. Der Führungsstift 47 ist mit seinem Rastzapfen 56 in der Durchgangsbohrung 34 des distalen Kupplungselementes 29 eingerastet.

So ist aus Fig. 9 erkennbar, dass das Sicherungselement 45 über den Führungsstift 47 sowie die Axialdruckfeder 46 feststehend mit dem Stanzschieber 8 in Verbindung steht. Aufgrund des formschlüssigen verschiebbaren Eingriffs seiner beiden Formschlusselemente 51 und 52, von welchen in Fig. 9 lediglich das Formschlusselement 52 sichtbar ist, mit den beiden Längsnuten 40 und 41, von welchen in Fig. 9 lediglich die hintere Längsnut 41 der hinteren Seitenwand 26 erkennbar ist, ist somit der Stanzschieber 8 über das Sicherungselement 45 unverlierbar mit dem Grundkörper 2 der Knochenstanze 1 verbunden. Wie aus Fig. 1 ersichtlich ist, weisen der Grundkörper 2 im Verbindungsbereich 5, insbesondere im Bereich der oberen Führungsfläche 13 und der Stanzschieber 8 im Bereich seiner Gleitfläche 14, im distalen Endbereich zumindest annähernd die gleiche Breite auf, so dass der Stanzschieber 8 in der in Fig. 9 dargestellten Position nicht in Richtung des Pfeiles 12 verstellt werden kann und somit der unverlierbare Eingriff des

Sicherungselementes 45 mit seinen beiden Formschlusselementen 51 und 52 mit den Längsnuten 40 und 41 nicht auflösbar ist. Da die Längsnuten 40 und 41 distal begrenzt sind, kann auch das Sicherungselement 45 mit seinen beiden Formschlusselementen 51 und 52 nicht entgegen des Pfeiles 12 aus den beiden Längsnuten 40 und 41 heraus geschoben werden.

Soll der Stanzschieber 8 vollständig vom Grundkörper 2 gelöst werden, so kann, beispielsweise mit einem entsprechenden Hilfswerkzeug, der Führungsstift 47 mit seinem Rastzapfen 56 aus der Durchgangsbohrung 34 durch einfaches Verstellen längs des Stanzschiebers 8 ausgerastet werden, so dass der Stanzschieber 8 in einfacher Weise vom Sicherungselement 45 und somit vom Grundkörper 2 abgenommen werden kann.

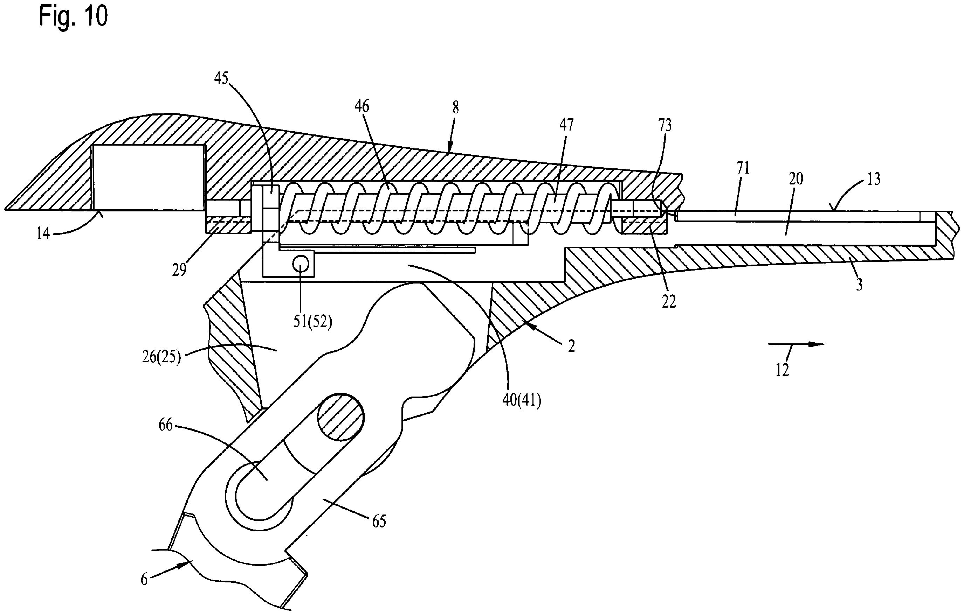

Um den Stanzschiebers 8 in seine normale, in Fig. 1 dargestellte Betriebsstellung zu bringen, wird dieser zunächst aus der in Fig. 9 dargestellten Schwenkposition in Richtung des Pfeiles 70 um die Formschlusselemente 51, 52 verschwenkt, bis der Stanzschieber 8 mit seiner unteren Gleitfläche 14 mit der oberen Führungsfläche 13 des Grundkörpers 2 bzw. des Führungsschaftes 3 in Kontakt gelangt, wie dies aus Fig. 10 ersichtlich ist.

Es ist erkennbar, dass der Stanzschieber 8 mit seiner unteren Gleitfläche 14 eben auf der Führungsfläche 13 des Grundkörpers 2 bzw. des Führungsschaftes 3 aufliegt. Des Weiteren ist aus Fig. 10 die Aufnahmenut 20 erkennbar. Mit Verweis auf Fig. 2 ist erkennbar, dass diese Aufnahmenut 20 T-förmig ausgebildet ist und beidseitig jeweils einen nach innen vorstehenden Gleitsteg 71 bzw. 72 aufweist, von welchen in Fig. 10 lediglich der Gleitsteg 71 erkennbar ist. Diese Gleitstege 71, 72 bilden distal jeweils eine Art Anschlag 73 bzw. 74, von welchen in Fig. 10 lediglich der Anschlag 73 erkennbar ist.

Wird nun der Stanzschieber 8 zusammen mit dem montierten Führungsstift 47, der Axialdruckfeder 46 sowie dem Sicherungselement 45 in Richtung des Pfeiles 12 verstellt (Fig. 10), so gelangt einerseits das Kupplungselement 22 mit der Aufnahmenut 20 in Eingriff und hintergreift aufgrund seiner T-förmigen Ausgestaltung die beiden zu Fig. 2 beschriebenen Gleitstege 71 und 72. Nach einem gewissen Stellweg in Richtung des Pfeiles 12 gelangt die Axialdruckfeder 46 mit ihrem proximalen Ende mit den beiden Anschlägen 73 und 74 der aus Fig. 2 erkennbaren Gleitstege 71 und 72 in Kontakt. Durch weiteres Verstellen des Stanzschiebers 8 relativ zum Grundkörper 2 in Richtung des Pfeils 12 werden zwangsläufig auch der Führungsstift 47, das Sicherungselement 45 sowie die beiden Kupplungselemente 22 und 29 in Richtung des Pfeiles 12 verstellt, wodurch eine Komprimierung der Axialdruckfeder 46 bewirkt wird, da diese mit ihrem proximalen Ende an den beiden Anschlägen 73, 74 ansteht. Gleichzeitig gleitet das Sicherungselement 45 mit seinen beiden Formschlusselementen 51 und 52 entlang der Längsnuten 40 und 41. Das Kupplungselement 29 gelangt aufgrund seiner T-förmigen Ausbildung mit den beiden Längsnuten 27 und 28 in Eingriff, so dass ein Abheben des Stanzschiebers 8 vom Grundkörper 2 nicht mehr möglich ist.

Nachdem der Stanzschieber 8 in seine "normale" Betriebsposition nach Fig. 1 gebracht wurde, kann der Betätigungshebel 6 mit seinem Stellhebel 65 über dessen Langloch 66 zwischen den beiden Seitenwänden 25 und 26 verstellt werden, wie dies aus der DE 20 2004 017 974 U1 bekannt ist.

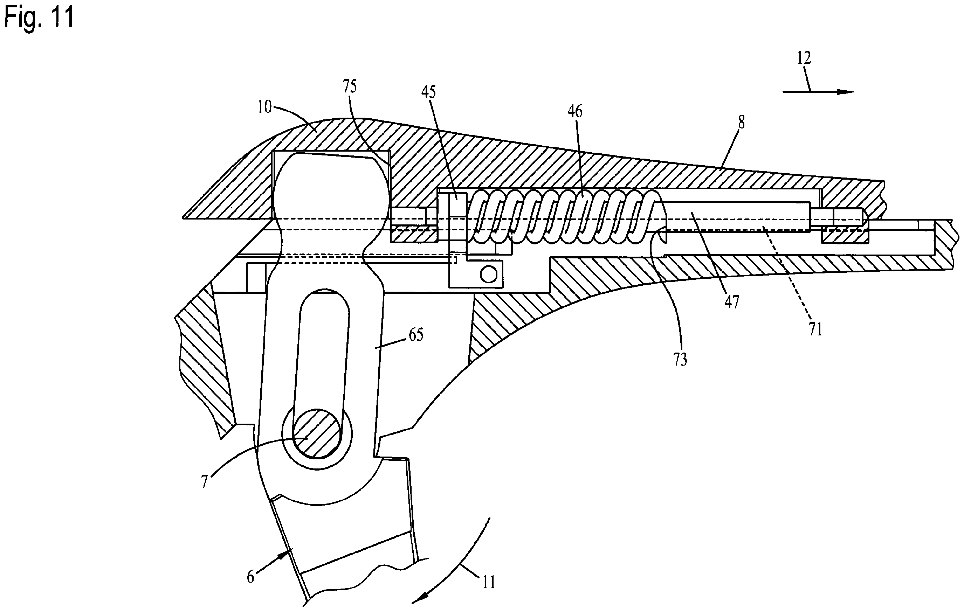

Dabei gelangt der Stellhebel 65 mit einer entsprechend unterseitig vorgesehenen Aussparung 75 im distalen Endbereich 10 des Stanzschiebers 8 in Eingriff, wie dies aus der Schnittdarstellung der Fig. 11 erkennbar ist.

Wird nun der Betätigungshebel 6 mit seinem Stellhebel 65 in Richtung des Pfeiles 11 um den Lagerzapfen 7 verschwenkt, so bewirkt dies eine Verstellung des Stanzschiebers 8 in Richtung des Pfeiles 12, wie dies aus dem Stand der Technik, insbesondere aus der DE 20 2004 017 974 U1, bekannt ist.

Bezüglich der sonstigen Funktionsweise der erfindungsgemäßen Knochenstanze wird ebenfalls auf diese Druckschrift DE 20 2004 017 974 U1 verwiesen, insbesondere was die Ausgestaltung des proximalen Endbereiches sowohl des Stanzschiebers 8 als auch des Führungsschaftes 3 betrifft.

An dieser Stelle sei noch angemerkt, dass prinzipiell die Konstruktion des Stanzschiebers 8 mit seinem Sicherungselement 45 auch einsetzbar ist, wenn keine Axialdruckfeder 46 mit Führungsstift 47 vorhanden ist. So ließe sich diese Konstruktion beispielsweise auch auf den Gegenstand der DE 10 2009 056 099 B4 prinzipiell anwenden.

Die lösbare Montage des Sicherungselementes 45 könnte dabei am distalen Kupplungselement 29 beispielsweise durch eine entsprechend angeordnete Verschraubung erfolgen, so dass das Sicherungselement 45 auch in einem solchen Fall lösbar mit dem Stanzschieber 8 in Verbindung steht (in der Zeichnung nicht dargestellt). Diese Konstruktion wäre allerdings im Bezug auf den Montagevorgang des Stanzschiebers am Sicherungselement etwas komplizierter, jedoch grundsätzlich möglich.

Vorteilhaft an der erfindungsgemäßen Ausgestaltung der Knochenstanze 1 ist, dass insbesondere das Sicherungselement 45 im normalen Betriebszustand der Knochenstanze 1 vollständig innerhalb des Stanzschiebers 8 und des Grundkörpers 2 der Knochenstanze 1 angeordnet ist und somit gegen äußere Einflüsse geschützt ist. Im normalen Betrieb der Knochenstanze 1 bleibt das Sicherungselement 45 stets vollständig von außen unzugänglich im Stanzschieber 8 bzw. dem Grundkörper 2, so dass insbesondere beim Betätigen der Knochenstanze 1 keinerlei Verletzungsgefahr für die Bedienungsperon besteht.

Somit ist die erfindungsgemäße Knochenstanze 1 mit ihrem Sicherungselement 45 und den zugehörigen Längsnuten 40, 41 in gleicher Weise einfach handhabbar wie die Knochenstanze aus der DE 20 2004 017 974 U1. Da das Sicherungselement 45 und die zugehörigen Längsnuten 40 und 41 vollständig innerhalb des Stanzschiebers 8 bzw. des Grundgehäuses 2 angeordnet sind, besteht hier auch nicht die Gefahr von Verunreinigungen dieser Bauteile während des Einsatzes.

Wie oben beschrieben, kann der Stanzschieber 8 auch in einfacher Weise, insbesondere durch die Verwendung des Führungsstiftes 47 zusammen mit der Axialdruckfeder 46, von diesen beiden Bauteilen getrennt werden, so dass hier automatisch und zwangsläufig auch eine Trennung vom Sicherungselement 45 erfolgt, wie oben beschrieben.

Somit lässt sich die erfindungsgemäße Knochenstanze 1 mit dem Sicherungselement 45 auch in einfacher Weise bei Bedarf vollständig zerlegen und auch wieder zusammenfügen.

## Patentansprüche

1. Knochenstanze (1) mit einem Grundkörper (2), der aus einem Führungsschaft (3) und einem am Führungsschaft (3) feststehend und abgewinkelt angeordneten Handgriff (4) besteht, mit einem Stanzschieber (8), welcher auf einer dem Handgriff (4) gegenüber liegenden, oberen Führungsfläche (13) des Führungsschaftes (3) längs des Führungsschaftes (3) verstellbar aufliegt, und mit einem Betätigungshebel (6), welcher in einem Verbindungsbereich (5) des Handgriffes (4) zum Führungsschaft (3) zwischen zwei voneinander beabstandeten Seitenwänden (25, 26) schwenkbar gelagert ist, wobei der Betätigungshebel (6) einen Stellhebel (65) bildet, über welchen der Betätigungshebel (6) mit dem Stanzschieber (8) zu dessen Verstellung längs des Führungsschaftes (3) entgegen der Federkraft einer Rückstellfeder (46) in Wirkverbindung steht, und wobei der Stanzschieber (8) über T-förmig profilierte, formschlüssig ineinander greifende und durch Längsverschiebung lösbare Führungselemente (15, 16, 20, 22, 27, 28, 29) am Führungsschaft (3) längs verstellbar geführt ist,
**dadurch gekennzeichnet,**
**dass** der Stanzschieber (8) in seinem distalen Endbereich (10) ein Sicherungselement (45) aufweist, welches im montierten Zustand des Stanzschiebers (8) auf dem Führungsschaft (3) zwischen die beiden Seitenwände (25, 26) hineinragt, dass das Sicherungselement (45) zwei seitlich vorstehende Formschlusselemente (51, 52) aufweist und
**dass** jede der beiden Seitenwände (25, 26) mit einer inneren in Längsrichtung (9) des Führungsschaftes (5) verlaufenden, distal begrenzten Längsnut (40, 41) versehen ist, in welche jeweils eines der Formschlusselemente (51, 52) längs verschiebbar einsetzbar ist.

2. Knochenstanze nach Anspruch 1, **dadurch gekennzeichnet, dass** das Sicherungselement (45) einen sich zum Stanzschieber (8) hin erstreckenden Lagerabschnitt (48) aufweist, mit welchem das Sicherungselement (45) lösbar mit dem Stanzschieber (8) in Verbindung steht.

3. Knochenstanze nach Anspruch 2, **dadurch gekennzeichnet, dass** als Rückstellfeder eine Axialdruckfeder (46) vorgesehen ist, welche auf einem Führungsstift (47) angeordnet ist und im montierten Zustand teilweise in einer unteren Aufnahmenut (32) des Stanzschiebers (8) angeordnet ist, wobei der Führungsstift (47) im montierten Zustand proximal in eine Aufnahmebohrung (33) des Stanzschiebers (8) und distal in eine zweite Bohrung (34) des Stanzschiebers (8) abnehmbar eingesetzt ist und
dass sich die Axialdruckfeder (46) bei vom Führungsschaft (3) abgenommenem Stanzschieber (8) proximal an einem ersten Anschlag (22) des Stanzschiebers (8) axial abstützt und dass sich die Axialdruckfeder (46) mittelbar oder unmittelbar an einem distalen zweiten Anschlag (29) des Stanzschiebers (8) axial abstützt und
dass das Sicherungselement (45) mit seinem Lagerabschnitt (48) zwischen dem distalen Ende der Axialdruckfeder (46) und dem zweiten Anschlag (29) aufgenommen ist.

4. Knochenstanze nach Anspruch 3, **dadurch gekennzeichnet, dass** der Führungsstift (47) in seinem distalen Endbereich einen radial vorstehenden Anschlagbund (54) aufweist, über welchen sich die Axialdruckfeder (46) mittelbar am zweiten, distalen Anschlag (29) axial abstützt und dass das Sicherungselement (45) mit seinem Lagerabschnitt (48) zwischen dem distalen Ende der Axialdruckfeder (46) und dem Anschlagbund (54) des Führungsstiftes (47) auf dem Führungsstift (47) aufgenommen ist.

5. Knochenstanze nach Anspruch 3 oder 4, **dadurch gekennzeichnet, dass** der erste Anschlag Teil eines ersten T-förmig ausgebildeten Kupplungselementes (22) des Stanzschiebers (8) ist, welches zur Führungsfläche (13) des Führungsschaftes (3) hin über die Gleitfläche (14) des Stanzschiebers (8) hinaussteht und im montierten Zustand mit einer T-förmig ausgebildeten Aufnahmenut (20) des Führungsschaftes (3) längs verschiebbar in Eingriff steht und
dass der zweite, distale Anschlag Teil eines zweiten T-förmig ausgebildeten Kupplungselementes (29) des Stanzschiebers (8) ist, welches zur Führungsfläche (13) des Führungsschaftes (3) hin über die Gleitfläche (14) des Stanzschiebers (8) hinaussteht und im montierten Zustand mit inneren, seitlich in den Seitenwänden (25, 26) angeordneten Längsnuten (27, 28) längs verschiebbar in Eingriff steht und
dass die beiden Anschläge gleichzeitig auch Teil der Aufnahmenut (32) des Stanzschiebers (8) für die Axialdruckfeder (46) sind.

6. Knochenstanze nach Anspruch 5, **dadurch gekennzeichnet, dass** der Stanzschieber (8) im Bereich seines ersten, proximalen Kupplungselementes (22) mit einer Aufnahmebohrung (33) versehen ist, in welche der Führungsstift (47) mit einem proximalen, radial verjüngt ausgebildeten Steckzapfen (55) einsteckbar ist und
dass der Stanzschieber (8) im Bereich seines zweiten, distalen Kupplungselementes (29) eine Durchgangsbohrung (34) aufweist und
dass der Führungsstift (47) in distaler, axialer Verlängerung zu seinem Anschlagbund (54) einen radial verjüngt ausgebildeten Rastzapfen (56) aufweist, mit welchem der Führungsstift (47) durch eine Axialverstellung des Führungsstiftes (47) in der Aufnahmebohrung entgegen der Federkraft der auf den Führungsstift (47) aufgesetzten Axialdruckfeder (46) mit der Durchgangsbohrung (34) im Bereich des zweiten Kupplungselementes (29) formschlüssig in Eingriff bringbar ist.

## Claims

1. Bone punch (1) having a main body (2), which consists of a guide shaft (3) and a handle (4), which is fixed in an angled state on the guide shaft (3),
having a punching slider (8), which rests on an upper guide surface (13) of the guide shaft (3), said upper surface being located opposite the handle (4), such that it can be displaced along the guide shaft (3),
and having an actuating lever (6), which is mounted in a region (5) connecting the handle (4) and the guide shaft (3) such that it can be pivoted between two spaced-apart side walls (25, 26), wherein the actuating lever (6) forms an adjusting lever (65), via which the actuating lever (6) is in operative connection with the punching slider (8) in order to adjust the latter along the guide shaft (3) counter to the force of a restoring spring (46),
and wherein the punching slider (8) is guided in a longitudinally adjustable manner on the guide shaft (3) via guide elements (15, 16, 20, 22, 27, 28, 29) which have a T-shaped profile, engage in a form-fitting manner one inside the other and can be released by longitudinal displacement,
**characterized**
**in that**, in its distal end region (10), the punching slider (8) has a securing element (45), which, in the assembled state of the punching slider (8) on the guide shaft (3), projects between the two side walls (25, 26),
**in that** the securing element (45) has two laterally projecting form-fitting elements (51, 52), and
**in that** each of the two side walls (25, 26) is provided with an inner, distally limited longitudinal groove (40, 41), which runs in the longitudinal direction (9) of the guide shaft (5) and into which a respective one of the form-fitting elements (51, 52) can be inserted in a longitudinally displaceable manner.

2. Bone punch according to Claim 1, **characterized in that** the securing element (45) has a bearing portion (48), which extends in the direction of the punching slider (8) and with which the securing element (45) is connected in a releasable manner to the punching slider (8).

3. Bone punch according to Claim 2, **characterized in that** the restoring spring provided is an axial compression spring (46) which is arranged on a guide pin (47) and, in the assembled state, is arranged to some extent in a lower accommodating groove (32) of the punching slider (8), wherein, in the assembled state, the guide pin (47) has been inserted in a removable manner, proximally, into an accommodating bore (33) of the punching slider (8) and, distally, into a second bore (34) of the punching slider (8), and
**in that**, with the punching slider (8) removed from the guide shaft (3), the axial compression spring (46) is axially supported, proximally, on a first stop (22) of the punching slider (8), and
**in that** the axial compression spring (46) is axially supported directly or indirectly on a second, distal stop (29) of the punching slider (8), and
**in that** the securing element (45) has its bearing portion (48) accommodated between the distal end of the axial compression spring (46) and the second stop (29).

4. Bone punch according to Claim 3, **characterized in that**, in its distal end region, the guide pin (47) has a radially projecting stop collar (54), via which the axial compression spring (46) is axially supported indirectly on the second, distal stop (29), and **in that** the securing element (45) is accommodated on the guide pin (47) with its bearing portion (48) between the distal end of the axial compression spring (46) and the stop collar (54) of the guide pin (47).

5. Bone punch according to Claim 3 or 4, **characterized in that** the first stop is part of a first T-shaped coupling element (22) of the punching slider (8), said coupling element projecting beyond the sliding surface (14) of the punching slider (8) in the direction of the guide surface (13) of the guide shaft (3) and, in the assembled state, engaging in a longitudinally displaceable manner with a T-shaped accommodating groove (20) of the guide shaft (3), and
**in that** the second, distal stop is part of a second T-shaped coupling element (29) of the punching slider (8), said coupling element projecting beyond the sliding surface (14) of the punching slider (8) in the direction of the guide surface (13) of the guide shaft (3) and, in the assembled state, engaging in a longitudinally displaceable manner with inner longitudinal grooves (27, 28) arranged laterally in the side walls (25, 26), and
**in that** the two stops, at the same time, are also part of the accommodating groove (32) of the punching slider (8) for the axial compression spring (46).

6. Bone punch according to Claim 5, **characterized in that**, in the region of its first, proximal coupling element (22), the punching slider (8) is provided with an accommodating bore (33), into which the guide pin (47) can be inserted by way of a proximal, radially tapered insertion stub (55),
and
**in that**, in the region of its second, distal coupling element (29), the punching slider (8) has a through-bore (34), and
**in that**, in the distal, axial extension of its stop collar (54), the guide pin (47) has a radially tapered latching stub (56), by way of which the guide pin (47) can be brought into form-fitting engagement with the through-bore (34) in the region of the second coupling element (29) by virtue of the guide pin (47) being adjusted axially in the accommodating bore counter to the force of the axial compression spring (46) positioned on the guide pin (47).

## Revendications

1. Kérisson rongeur (1) avec un corps de base (2) composé d'une tige de guidage (3) et d'une poignée (4) disposée fixement et de façon coudée au niveau de la tige de guidage (3) ;
avec un coulisseau d'estampage (8) reposant sur une surface de guidage (13), supérieure opposée à la poignée (4), de la tige de guidage (3), de façon à pouvoir être réglé dans le sens de la longueur de la tige de guidage (3) ; et
avec un levier d'actionnement (6) disposé dans une zone de liaison (5) de la poignée (4) avec la tige de guidage (3) de façon à pouvoir pivoter entre deux parois latérales (25, 26) placées à une certaine distance l'une de l'autre, le levier d'actionnement (6) formant un levier de réglage (65) via lequel le levier d'actionnement (6) est en liaison active avec le coulisseau d'estampage (8) pour son réglage dans le sens de la longueur de la tige de guidage (3) à l'encontre de la force de ressort d'un ressort de rappel (46) ;
et le coulisseau d'estampage (8) étant guidé de façon à pouvoir être réglé dans le sens de la longueur au niveau de la tige de guidage (3) via des éléments de guidage (15, 16, 20, 22, 27, 28, 29) profilés en forme de T s'engrenant par complémentarité de formes les uns dans les autres et pouvant être détachés par coulissement longitudinal ;
**caractérisé en ce que** :
le coulisseau d'estampage (8) comporte dans sa zone d'extrémité distale (10) un élément de sécurité (45) rentrant, à l'état monté du coulisseau d'estampage (8), sur la tige de guidage (3), entre les deux parois latérales (25, 26) ; et
l'élément de sécurité (45) comporte deux éléments de complémentarité de formes (51, 52) saillant en côté ; et
chacune des deux parois latérales (25, 26) est pourvue d'une rainure longitudinale (40, 41) intérieure délimitée distalement s'étendant dans la direction longitudinale (9) de la tige de guidage (5) dans laquelle respectivement un des éléments de complémentarité de formes (51, 52) peut être inséré de façon à pouvoir être déplacé dans le sens de la longueur.

2. Kérisson rongeur selon la revendication 1, **caractérisé en ce que** l'élément de sécurité (45) comporte une section de palier de roulement (48) s'étendant en direction du coulisseau d'estampage (8) et à l'aide de laquelle l'élément de sécurité (45) est relié de façon amovible au coulisseau d'estampage (8).

3. Kérisson rongeur selon la revendication 2, **caractérisé en ce qu'**un ressort de pression axiale (46) servant de ressort de rappel est prévu, celui-ci étant disposé sur une tige de guidage (47) et étant disposé à l'état monté en partie dans une rainure de logement (32) inférieure du coulisseau d'estampage (8), la tige de guidage (47) étant insérée, à l'état monté, proximalement dans un alésage de logement (33) du coulisseau d'estampage (8) et distalement dans un deuxième alésage (34) du coulisseau d'estampage (8), de façon à pouvoir en être retirée ; et
le ressort de pression axiale (46) bute, en présence du coulisseau d'estampage (8) retiré de la tige de guidage (3), dans le plan axial, proximalement contre une première butée (22) du coulisseau d'estampage (8) et le ressort de pression axiale (46) bute dans le plan axial directement ou indirectement contre une deuxième butée (29) distale du coulisseau d'estampage (8) ; et l'élément de sécurité (45) est logé avec sa section de palier de roulement (48) entre l'extrémité distale du ressort de pression axiale (46) et la deuxième butée (29) .

4. Kérisson rongeur selon la revendication 3, **caractérisé en ce que** la tige de guidage (47) comporte dans sa zone d'extrémité distale une collerette de butée (54) saillant dans le plan radial via laquelle le ressort de pression axiale (46) bute de façon indirecte dans le plan axial contre la deuxième butée (29) distale et que l'élément de sécurité (45) est logé avec sa section de palier de roulement (48) sur la tige de guidage (47), entre l'extrémité distale du ressort de pression axiale (46) et la collerette de butée (54) de la tige de guidage (47).

5. Kérisson rongeur selon la revendication 3 ou 4, **caractérisé en ce que** la première butée fait partie d'un premier élément de couplage (22) réalisé en forme de T du coulisseau d'estampage (8) ressortant en direction de la surface de guidage (13) de la tige de guidage (3) par-delà la surface de glissement (14) du coulisseau d'estampage (8) et est en prise à l'état monté avec une rainure de logement (20) réalisée en forme de T de la tige de guidage (3) de façon à pouvoir être déplacée dans le sens de la longueur et que la deuxième butée distale fait partie d'un deuxième élément de couplage (29) réalisé en forme de T du coulisseau d'estampage (8) ressortant en direction de la surface de guidage (13) de la tige de guidage (3) par-delà la surface de glissement (14) du coulisseau d'estampage (8) et est en prise à l'état monté avec des rainures longitudinales (27, 28) intérieures disposées en côté dans les parois latérales (25, 26) de façon à pouvoir être déplacée dans le sens de la longueur ; et que les deux butées font simultanément également partie de la rainure de logement (32) du coulisseau d'estampage (8) pour le ressort de pression axiale (46) .

6. Kérisson rongeur selon la revendication 5, **caractérisé en ce que** le coulisseau d'estampage (8) est pourvu, dans la zone de son premier élément de couplage (22) proximal, d'un alésage de logement (33) dans lequel la tige de guidage (47) peut être enfichée avec un tenon d'enfichage (55) proximal réalisé de façon à se rétrécir dans le plan radial ; et
que le coulisseau d'estampage (8) comporte, dans la zone de son deuxième élément de couplage (29) distal, un alésage traversant (34) ; et
que la tige de guidage (47) comporte, dans le prolongement axial distal par rapport à sa collerette de butée (54), un tenon d'arrêt (56) réalisé de façon à se rétrécir dans le plan radial et avec lequel la tige de guidage (47) peut être amenée en prise par complémentarité de formes avec l'alésage traversant (34) dans la zone du deuxième élément de couplage (29) par le biais d'un déplacement axial de la tige de guidage (47) dans l'alésage de logement, à l'encontre de la force de ressort du ressort de pression axiale (46) placé sur la tige de guidage (47).
